# EUROPEAN PATENT SPECIFICATION

(11) **EP 3 150 235 B1**
(45) Date of publication and mention of the grant of the patent: **01.08.2018**
(21) Application number: 16195130.6
(22) Date of filing: 18.01.2013
(51) Int. Cl.: A61L 27/12, A61L 27/18, A61L 27/54, A61L 27/58, A61L 24/02

(54) **BONE FILLER COMPOSITION**
KNOCHENFÜLLMITTELZUSAMMENSETZUNG
COMPOSITION D'UN AGENT DE REMPLISSAGE OSSEUX

(30) Priority: 19.01.2012 GB 201200868
(43) Date of publication of application: 05.04.2017
(62) Divisional of application: 13702091.3
(73) Proprietor: Depuy International Limited, Leeds, West Yorkshire LS11 8DT (GB)
(72) Inventor: ALI, Saad Abdul Majeed, Leeds, West Yorkshire LS11 8DT (GB)
(74) Representative: Curran, Clair

(56) References cited:
- WO-A1-02/080933

## Description

This invention relates to a curable calcium phosphate based bone filler which includes a bisphosphonate formulation.

WO-A-02/080933 discloses a bone graft material which can be based on a calcium hydroxyapatite or tricalcium phosphate and which can include at least one bisphosphonate.

It is known that bisphosphonates can be used in the treatment of osteoporosis. Bisphosphonates act by binding to the surface of a bone resulting in a reduction in the rate of bone resorption. It is believed that bisphosphonates suppress the migration of osteoclast pre-cursors on to the surface of a bone and consequently the formation on the bone surface of active resorbing osteoclasts. It has also been suggested that bisphosphonates can cause increased osteoblast formation (see the paper by Reinholz G G et al entitled "Bisphosphonates directly regulate cell proliferation, differentiation, and gene expression in human osteoblasts" published in Cancer Research, vol. 60, 6001-6007, 2000).

WO-A-02/062352 discloses a device for delivery of a bisphosphonates for the purpose of reducing the rate of bone resorption. The bisphosphonate is provided in a sustained release dosage form which can function as an implantable depot from which the drug can be released into the patient's circulation. Alternatively, the dosage form can be implanted at a site which is close to the desired site of action so that the drug, when released, can reach the site of action by diffusion. The drug can be suspended in a liquid, or it can be integrated into a polymer matrix.

Calcium phosphate based bone filler materials have been proposed for use in the treatment of osteoporotic patients. The materials can be injected into a vertebral body where it hardens so that the body is augmented. WO-A-02/062351 relates to the use of bisphosphonates in the treatment of osteonecrosis; osteonecrosis can lead to the formation of osteoporotic bone tissue. The document discloses direct application to bone surfaces of a bone graft substitute which can contain tri-calcium phosphate in combination with an effective amount of a bisphosphonate, and a resorbable organic material as a carrier medium.

It has been found that incorporation of bisphosphonate particles in a calcium phosphate based bone filler can affect the rate of cure of the bone filler.

The present invention provides a formulation for admixture with a curable calcium phosphate based bone filler, which comprises a bisphosphonate in particulate form, the particles of the bisphosphonate being embedded in particles of a polymeric material which resorbs when the formulation is implanted.

The formulation of the invention has the advantage that it can confer properties on a calcium phosphate based bone filler which mean that the filler can be used in the treatment of osteoporosis. Furthermore, it has been found the provision of bisphosphonate particles embedded in particles of a resorbable polymeric material can help to reduce adverse effects on the rate of a calcium phosphate bone filler as it cures by reacting in a hardening reaction. It can mean that the time taken from the mixing of the bone filler components for the filler, with the encapsulated bisphosphonate particles, to reach a condition in which it can be injected is not significantly longer than the corresponding time taken for a filler without the encapsulated bisphosphonate particles to reach the same condition. It can mean that the duration of the period in which the filler with the encapsulated bisphosphonate particles can be injected or otherwise manipulated is not significantly shorter than the corresponding period for a filler without the encapsulated bisphosphonate particles. These represent significant practical advantages for a surgeon.

The formulation of the invention has the advantage that the mechanical properties of a calcium phosphate based bone filler composition (for example one or more of its tensile strength, compressive strength, and toughness (resistance to fracture)) containing a bisphosphonate which is embedded in polymer can be improved compared with the corresponding properties of a calcium phosphate based bone filler composition containing a bisphosphonate which is not embedded in polymer. In some circumstances, it is expected that the mechanical properties of the composition of the invention can be at least comparable with those of a calcium phosphate based bone filler composition which does not contain bisphosphonate.

The bisphosphonate class of drugs is based on the phosphate-oxygen-phosphate bond (P-O-P) of pyrophosphate (a widely distributed, natural human metabolite that has a strong affinity for bone). Replacing the oxygen with a carbon atom (P-C-P) produces a group of bone-selective drugs that cannot be metabolized by the normal enzymes that break down pyrophosphates (see the paper by Gatti D and Adami S entitled "New bisphosphonates in the treatment of bone diseases", published in Drugs & Aging, vol. 15, pages 285 to 296, 1999). Bisphosphonates which are useful in the present invention generally have anti-catabolic characteristics. Examples of bisphosphonate compounds are disclosed in US-6090410, US-6008207, US-6008206, US-5994329, US-5958908, US-5854227, US-5849726, US-5804570, US-5681590, US-5583122, US-5574024, US-5431920, US-5358941, US-5356887, US-5344825, US-5270365, US-5237094, US-5227506, US-5183815, US-5070108, US-5041428, US-4980171, US-4963681, US-4942157, US-4927814, US-4922007, US-4876248, US-4711880, US-4621077, US-4267108 and US-4054598.

Specific examples of bisphosphonate compounds which might be useful in the formulation of the invention include elendronate (4-amino-1-hydroxybutylidene)bisphosphonate (Gentili, Merck Sharp & Dohme), etidronate(1-hydroxyethylidene)bisphosphonate (Gentili; Procter & Gamble), clodronate(dichlorormethylene)bisphosphonate (Astra; Boehringer Mannheim; Gentili; Leiras; Rhone-Poulenc Rorer), tiludronate[[(4-chlorophenyl)thio]-methylene]bisphosphonate (Sanofi), pamidronate (3-amino-1-hydroxy-propand-1,1-diyl) bisphosphonate (Ciba-Geigy; Gador), neridronate (6-amino-1-hydroxy-hexylidene)bisphosphonate (Gentili), cimadronate[(cycloheptylamino)-methylene] bisphosphonate (Yamanouchi), EB-1053 [1-hydroxy-3-(1-pyrrolidinyl)-propylidene] bisphosphonate (Leo), olpadronate [3-(dimethylamino)-1-hydroxypropylidene] bisphosphonate (Gador), ibandronate [1-hydroxy-3-(methylpentylamino)propylidene] bisphosphonate (Boehringer Mannheim), risedronate([1-hydroxy-2-(3-pyridinyl)-ethylidene]bisphosphonate (Procter & Gamble), YH 529 [1-hydroxy-2-imidazo-(1,2-a)-pyridin-3-yl ethylidene] bisphosphonate (Yamanouchi), and zoledronate[1-hydroxy-2-(1H-imidazol-1-yl)-ethylidene]bisphosphonate (Ciba-Geigy).

The advantages that are available from the formulation of the invention are affected by the ability of the polymeric material to mask the effect of the bisphosphonate on the calcium phosphate material as it cures. The polymeric material should be essentially insoluble in aqueous media over the period in which the calcium phosphate material cures. The polymeric material should be capable of resorbing over a sustained period after the formulation of the invention has been implanted so that the polymeric material disappears, whether as a result of dissolution or as a result another mechanism or as a result of a combination of mechanisms. Resorption of the polymeric material can involve dissolution. Resorption of the polymeric material can involve a reaction with materials with which it comes into contact when the formulation is implanted, for example involving hydrolysis. Such hydrolysis might result in cleavage of chains of the polymeric material.

It can be preferred that the polymeric material has hydrophobic characteristics. Such materials are characterised by a water contact angle of at least 90°.

The polymeric material can comprise a monomer which has hydrophobic properties and a monomer which has hydrophilic properties. Such materials are characterised by a water contact angle of not more than 90°.

The polymeric material can include chain terminating groups which are different from the repeat units from which the polymer chains are made up. The chain terminating groups can affect interactions between the polymeric material and other components of the formulation. The chain terminating groups can affect interactions between the polymeric material and materials with which the formulation comes into contact when the formulation has been implanted. For example, when the repeat units from which the polymer chains are made up have hydrophobic characteristics, the chains can include terminating groups which have a more hydrophilic character. This can be used for example to enhance the interactions between the polymeric material and bisphosphonate particles which are embedded in the polymeric material. An example of a hydrophilic terminating group is an acid group. The polymer can be terminated by a group having hydrophobic character. Such a terminating group can be an ester group.

The polymeric material can include a lactide polymer. The polymer can include lactide groups in a copolymer, for example as a lactide/glycolide copolymer or with ε-caprolactone or δ-valerolactone. The lactide can be the D-enantiomer. The lactide can be the L-enantiomer. The lactide can include the D-enantiomer and the L-enantiomer.

The polymeric material can include a glycolide polymer.

Specific examples of polymeric materials which might be useful in the formulation of the invention include glycolide/lactide copolymers (PGA/PLA), poly-L-lactide (PLLA), poly-D-lactide (PDLA), poly-DL-lactide (PDLLA), L-lactide/D-lactide copolymers, L-lactide/DL-lactide copolymers, lactide/ε-caprolactone copolymers, and lactide/δ-valerolactone copolymers.

A polymeric material which is based on a lactide polymer or a lactide/glycolide copolymer can be terminated with acid groups. Such terminating groups can have hydrophilic character. Acid-terminated polymers can be reacted with another species in a nucleophilic substitution reaction. This can help to increase the resistance of the polymer to resorption processes. For example, an acid-terminated polymer can be reacted with an alcohol in order to form an ester-terminated polymer. The resulting terminating groups can have hydrophobic character. The susceptibility of the polymer to resorption processes can be affected by the molecular weight of the substituting group (for example, the alcohol when the polymer end product is an ester-terminated polymer). It can be preferred that an alcohol which is used as the substituting group is not bigger than C₅, more preferably not bigger than C₄, especially not bigger than C₃, for example ethanol.

The ability of the polymeric material to resorb after implantation can depend on the crystallinity of the polymeric material. Polymer crystallinity can be measured using differential scanning calorimetry. Polymers with a relatively high crystallinity are resorbed more slowly than polymers with a relatively low crystallinity. It can be preferred in the present invention to use polymeric materials which have a low crystallinity so that they can be resorbed over a suitably short period after the formulation has been implanted. However, polymers which have a higher crystallinity (for example including at least some poly(L-lactide)) can be used in useful formulations which are intended use in applications in which the bisphosphonate is released over a longer period.

The ability of the polymeric material to resorb after implantation can depend on the molecular weight of the polymeric material. A formulation which is based on a polymeric material with a relatively high molecular weight will release bisphosphonate over a period that is longer than the corresponding period when a polymeric material with a lower molecular weight is used. It can be preferred that the polymeric material has a molecular weight of at least about 10 kD, more preferably at least about 15 kD, especially at least about 20 kD. It can be appropriate for some applications to have a molecular weight of at least about 25 kD for some applications. Generally the polymeric material will have a molecular weight of not more than about 150 kD, preferably not more than about 140 kD, especially not more than about 130 kD. Molecular weight values referred to in this document are weight average molecular weights.

It can be preferred that the polymeric material is a lactide polymer or a lactide/glycolide copolymer, for example a (D,L) lactide/glycolide copolymer, or a poly(D,L) lactide. Glycolic acid polymers are partially amorphous and partially crystalline (semi-crystalline). They tend to be hydrophilic. Lactide acid homopolymers are semi-crystalline and hydrophobic. The polymeric material should preferably be hydrophobic. The material should preferably be amorphous or semi-crystalline. The polymeric material should preferably have a molecular weight of at least about 15 kD. The polymeric material should preferably have a molecular weight of not more than about 200 kD, more preferably no more than about 175 kD, especially not more than about 150 kD, for example not more than about 140 kD. Many preferred materials have a molecular weight of not more than about 130 kD.

Preferably the inherent viscosity midpoint of a polymeric material which is a lactide polymer or a lactide/glycolide copolymer is at least about 0.10 dl.g⁻¹, more preferably at least about 0.15 dl.g⁻¹. Preferably, the inherent viscosity midpoint of a polymeric material which is a lactide polymer or a lactide/glycolide copolymer is not more than about 6.0 dl.g⁻¹, more preferably not more than about 4.5 dl.g⁻¹, for example not more than about 3.0 dl.g⁻¹, or not more than about 1.50 dl.g⁻¹, or not more than about 1.0 dl.g⁻¹, or not more than about 0.60 dl.g⁻¹. Inherent viscosity can be measured for these polymers using a 1.0 g.dl⁻¹ solution of the polymer in CHCl₃ in a capillary viscometer at 25°C.

A suitable polymeric material for use in the formulation of the invention is an acid terminated poly-DL-lactide with an inherent viscosity midpoint of at least about 0.20 dl.g⁻¹. An example of this material is available from the Purac division of CSM N V under the trade mark PURASORB PDL-02A.

A suitable polymeric material for use in the formulation of the invention is an ester terminated poly-DL-lactide with an inherent viscosity midpoint of at least about 0.50 dl.g⁻¹. An example of this material is available from the Purac division of CSM N V under the trade mark PURASORB PDL-05.

Suitable copolymers for use in the formulation of the invention are lactide/glycolide copolymers. Preferably, the value of the molar ratio of (D,L) lactide and glycolide in suitable copolymers is at least about 0.75, especially at least about 0.9, for example at least about 1.0. Preferably, the value of the molar ratio is not more than about 4.5, more preferably not more than about 4.0, for example not more than about 3.5.

Examples of suitable acid terminated copolymer materials are available from the Purac division of CSM N V under the trade marks PURASORB PDLG-5002A, PDLG-5004A and PDLG-7502A. Characteristics of these materials are set out in the following table.

| | PDLG-5002A | PDLG-5004A | PDLG-7502A | PDL-02A |
|---|---|---|---|---|
| DL-lactide content (mol %) | 47-53 | 47-53 | 72-78 | 100 |
| Glycolide content (mol %) | 53-47 | 53-47 | 28-22 | 0 |
| Inherent viscosity (dl.g⁻¹) | 0.16-0.24 | 0.32-0.48 | 0.16-0.24 | 0.16-0.24 |

Examples of suitable ester terminated copolymer materials arc available from the Purac division of CSM N V under the trade marks PURASORB, PDLG-5004, PDLG-5010 and PDLG-7507. Characteristics of these materials are set out in the following table.

| | PDLG-5004 | PDLG-5010 | PDLG-7507 | PDL-05 |
|---|---|---|---|---|
| DL-lactide content (mol %) | 47-53 | 47-53 | 72-78 | 100 |
| Glycolide content (mol %) | 53-47 | 53-47 | 28-22 | 0 |
| Inherent viscosity (dl.g⁻¹) | 0.32-0.48 | 0.84-1.24 | 0.61-0.91 | 0.4-0.6 |

Varying the ratio of lactide and glycolide components of a copolymer can be used to provide control over the rate at which the polymer is resorbed after implantation.

It can be preferred that the weight proportion of the bisphosphonate expressed as a proportion of the weight of the formulation is not more than about 30%, more preferably not more than about 25%, especially not more than about 20%, for example not more than about 15%. It can be preferred that the weight proportion of the bisphosphonate expressed as a proportion of the weight of the formulation is at least about 0.5%, more preferably at least about 1.0%, especially at least about 1.5%.

The particles of the polymeric material in which the bisphosphonate particles are embedded can have a low aspect ratio. Low aspect ratio particles have a small difference between their largest and smallest transverse dimensions. Frequently, the particles of the polymeric material will be approximately spherical. Spherical particles have an aspect ratio of one. Particles of the polymeric material which have an elliptical shape have an aspect ration which is greater than one. The particles of the invention can have an aspect ratio which is not more than about 3, or not more than about 2, or not more than about 1.5. Such particles can be formed using emulsification techniques. Information concerning emulsification techniques is included in the paper by Nafea et al, Alendronate PLGA microspheres with high loading efficiency for dental applications (Journal of Microencapsulation, 2007, Vol 24(6), pp 525-528). Suitable techniques include single emulsion techniques such as solid-oil-water techniques, and double emulsion techniques such as water-oil-oil techniques. A water-oil-oil technique involves emulsification of an internal aqueous solution of the drug in an organic phase O₁ consisting of the polymer dissolved in a binary solvent system. The primary W/O₁ emulsion was emulsified into a non-aqueous processing medium O₂ containing an emulsifier to form a W-O₁-O₂ emulsion. Solvents were removed by evaporation while stirring the emulsion overnight. Variable volume ratio of W-O₁-O₂ phases was used. The polymer solution concentration used was 6.25% w/v. The use of water-oil-oil techniques can have advantages when it is desired to encapsulate a water-soluble drug in a polymer because it can increase the efficiency with which the drug is entrapped in the particles.

The formulation of the invention can be made using a melt processing technique in which the drug is mixed with the polymer while the polymer is in a fluid phase. This process can be performed by exposing a mixture of particles of the polymer and particles of the drug to heat to cause the polymer to melt. The process can be performed by adding particles of the drug to the polymer after the polymer has been made to melt. The mixture of the polymer and the drug particles is then allowed to harden. The method can include a step of shaping the mixture of the polymer and the drug particles. For example, this might be done by extruding the mixture of the polymer and the drug particles. This technique can be used to create particles of the polymeric material which are elongate, for example in the form of fibres. Fibres produced by extrusion can be modified by subsequent processing steps such as for example stretching, spinning, preferably while the fibres are heated. Elongate particles can have the advantage that they can help to reinforce a bone filler material in which the formulation of the invention is mixed. It can be preferred that the transverse dimension of elongate particles is at least about 0.1 mm, more preferably at least about 0.5 mm, for example at least about 1.0 mm. It can be preferred that the transverse dimension of elongate particles is not more than about 5.0 mm, for example not more than about 3.0 mm. It can be preferred that the length of elongate particles is at least about 1.0 mm, more preferably at least about 2.0 mm, for example at least about 3.0 mm. It can be preferred that the length of elongate particles is not more than about 25 mm, more preferably not more than about 15 mm, especially not more than about 10 mm, for example not more than about 7 mm.

Accordingly, it can be preferred that the particles of the polymeric material are in the form of fibres. Fibres have a generally constant cross-section along their length. Fibres will frequently have a length which is at least twice the average transverse dimension (which will be the diameter of the fibres when their cross-section is circular). It can be preferred for some applications that the value of the ratio of the length of the fibres to their average transverse dimension is at least about 1.5, or at least about 2.0, or at least about 2.5, for example at least about 3.0.

Factors affecting the choice of the size of the polymer particles include the rate of release of the drug from the polymer particles and the effect on the physical properties of the polymer particles. The period over which drug might be released from polymer particles can be increased by use of larger particles because the drug is less accessible to body fluids.

The use of a melt processing technique to form the particles has the advantage that the rate of release of the drug from the particles can be compared with particles which are made from an emulsion. This can be because of a lower porosity.

It can be preferred that 90% by weight of the particles of the bisphosphonate which are embedded in the particles of the polymeric material have a particle size (D90) of not more than about 50 µm, more preferably not more than about 30 µm, especially not more than about 25 µm. The use of small particles of the bisphosphonate can facilitate encapsulation of the drug particles by the polymeric material.

It can be preferred that 90% by weight of the particles of the polymeric material in which the bisphosphonate particles are embedded have a particle size (D90) of not more than about 100 µm, more preferably not more than about 85 µm, especially not more than about 70 µm. Preferably 90% by weight of the particles of the polymeric material will usually have a particle size (D90) of at least about 50 µm.

It can be preferred that the formulation is used with a calcium phosphate powder in which 90% by weight of the particles of the calcium phosphate powder have a particle size (D90) which is not more than about 75 µm, preferably not more than about 50 µm, more preferably not more than about 30 µm, especially not more than about 25 µm. The use of calcium phosphate powder with a small particle size can help to provide a cured bone filler with desirable mechanical properties.

It can be preferred that the formulation is used with a calcium phosphate powder in which the ratio of the D90 particle size of the calcium phosphate powder to the D90 particle size of the particles of the polymeric material is at least about 0.1, preferably at least about 0.2, more preferably at least about 0.3, for example at least about 0.4. It can be preferred that the formulation is used with a calcium phosphate powder in which the ratio of the D90 particle size of the calcium phosphate powder to the D90 particle size of the particles of the polymeric material is not more than about 1.5, preferably not more than about 1.1. The use of calcium phosphate powder with a particle size which is similar to that of the particles of polymeric material can help to provide a cured bone filler with desirable mechanical properties.

It is preferred that the bisphosphonate particles are embedded in the polymeric material so that the surface area of the bisphosphonate particles that is exposed for contact with calcium phosphate is small and the bisphosphonate particles are almost or actually completely encapsulated in the polymeric material. It is expected that there might in some embodiments be some bisphosphonate which is exposed on the surface of the polymer particles and which might therefore contact calcium phosphate when the polymer/bisphosphonate particles are mixed with calcium phosphate powder.

It is preferred that the bisphosphonate particles are embedded in the polymeric material so that the bisphosphonate particles are at least partly covered by the polymeric material. It is envisaged that the bisphosphonate particles can have a coating of the polymeric material applied to them so that they are at least partly covered by the polymeric material. In these embodiments, the size of the bisphosphonate particles might only be slightly smaller than the particles of the polymeric material in which the bisphosphonate particles are embedded.

It is preferred that at least some of the bisphosphonate particles are completely embedded in the particles of the polymeric material so that those bisphosphonate particles are completely covered by the polymeric material.

The bisphosphonate particles can be embedded in the polymeric material with multiple particles of the bisphosphonate in each of the particles of the polymeric material and with each of the bisphosphonate particles in any particle of the polymeric material at least partly covcred, preferably fully covcred, by the polymeric material. This will frequently be the case when the size of the bisphosphonate particles is significantly smaller than the size of the particles of the polymeric material, for example when the bisphosphonate particles are prepared so that 90% by weight have a particle size which is not more than about 25 µm and the size of the polymer particles is at least about 50 µm. It will be understood that a sample of bisphosphonate particles is prepared so that 90% by weight have a particle size which is not more than about 25 µm, the sample will include a large proportion of particles whose size is significantly less than 30 µm. For example, a sample of bisphosphonate particles with a D90 particle size of not more than 25 µm might have the following size distribution:

| Max particle size (µm) | Proportion by weight (%) |
|---|---|
| 0.39 | 10 |
| 7.37 | 50 |
| 24.56 | 90 |

Accordingly, a polymer particle might include one or more bisphosphonate particles which are completely covered by the polymeric material and one or more bisphosphonate particles which are partly covered by the polymeric material.

The calcium phosphate based bone filler is based on the system Ca₃(PO₄)₂-H₃PO₄-H₂O which transforms from a liquid or pasty state to a solid state, where the end product of the reaction is a calcium phosphate. The system usually includes a concentrated mixture of one or more calcium phosphate powders and water or one or more aqueous solutions.

The calcium phosphate end product should be capable of resorption when the material is implanted. A suitable calcium phosphate end product is dicalcium phosphate dihydrate, referred to as brushite. This can be formed when the starting phosphate is β-tricalcium phosphate. The formation of brushite as the reaction product can be controlled by use of acidic conditions during the reaction.

An example of a reaction in which brushite is formed from a β-tricalcium phosphate starting product is:

β-Ca₃PO₄)₂ + H₃PO₄ + 6H₂O → 3CaHPO₄.2H₂O

Preferably, the powder component from which the calcium phosphate based bone filler is formed contains β-tricalcium phosphate in an amount of at least about 85% by weight based on the total weight of the powder component of the bone filler, more preferably at least about 90%, especially at least about 97.5%. The powder component might include other materials such as for example sodium pyrophosphate and hydroxyapatite (Ca₁₀(PO₄)₆(OH)₂).

Preferably, the brushite content in the cured bone filler material is at least about 50% by weight, expressed as a proportion of the total weight of the bone filler material (not including the bisphosphonate loaded polymer particles), more preferably at least about 60%, especially at least about 70%.

In another aspect, the invention includes a bone filler composition which comprises a mixture of a curable calcium phosphate based bone filler which is formed from a liquid component and a calcium phosphate based powder component, and a formulation as discussed above.

It can be preferred that the powder component of the bone filler comprises at least 50% by weight β-tricalcium phosphate, expressed as a percentage of the total weight of the powder component of the bone filler.

It can be preferred that the bisphosphonate loaded polymeric material particles are present in an amount of not more than about 60% by weight, more preferably not more than about 50% by weight, for example not more than 45%, or not more than 40%, expressed as a percentage of the total weight of powder component of the bone filler.

It can be preferred that the bisphosphonate loaded polymeric material particles are present in an amount of at least about 10% by weight, expressed as a percentage of the total weight of powder component of the bone filler. An amount of the bisphosphonate loaded polymeric material in the composition of at least about 10% can be especially appropriate when the particles are made by an emulsification technique.

It can be preferred that the bisphosphonate loaded polymeric material particles in the bone filler composition are present in an amount of not more than about 25% by weight, more preferably not more than about 20% by weight, for example not more than 15% by weight, expressed as a percentage of the total weight of powder component of the bone filler. An amount of the bisphosphonate loaded polymeric material in the composition of not more than 25% (or not more than a lower limit referred to above) can be especially appropriate when the particles are made by a melt processing technique when the amount of the bisphosphonate in the bisphosphonate loaded polymeric material particles is not limited by solubility of the bisphosphonate in a solvent.

It can be preferred that the bisphosphonate loaded polymeric material particles are present in the bone filler composition an amount of at least about 1% by weight, more preferably at least about 3% by weight, for example at least about 5%, expressed as a percentage of the total weight of powder component of the bone filler.

It can be preferred that the weight proportion of the bisphosphonate expressed as a proportion of the weight of the composition is not more than about 6%, more preferably not more than about 5%, for example not more than about 4%. It can be preferred that the weight proportion of the bisphosphonate expressed as a proportion of the weight of the formulation is at least about 0.01%, more preferably at least about 0.05%, especially at least about 1.0%, for example at least about 1.5%. An amount of the bisphosphonate in the composition of not more than 6% (or not more than a lower limit referred to above) can be especially appropriate when the particles are made by an emulsification technique.

### EXAMPLE 1 - Solid-oil-water emulsion method

### Preparation of particles

The following materials were used to manufacture particles:
- Acid terminated lactide/glycolide copolymer Purasorb PDLG-5004A (IV=0.41 dl.g⁻¹; Mw=53 kD) supplied by Purac.
- Acid terminated lactide/glycolide copolymer Purasorb PDLG-5002A (IV=0.21 dl.g⁻¹; Mw=20 kD) supplied by Purac.
- Acid terminated lactide/glycolide copolymer Purasorb PDLG-7502A (IV=0.18 dl.g⁻¹; Mw=17 kD) supplied by Purac.
- Acid terminated poly-DL-lactide Purasorb PDL-02A (IV=0.21 dl.g⁻¹; Mw=22 kD) supplied by Purac.
- Ester terminated lactide/glycolide copolymer Purasorb PDLG-5004 (IV=0.41 dl.g⁻¹; Mw=42 kD) supplied by Purac.
- Ester terminated lactide/glycolide copolymer Purasorb PDLG-5010 (IV=1.04 dl.g⁻¹; Mw=128 kD) supplied by Purac.
- Ester terminated lactide/glycolide copolymer Purasorb PDLG-7507 (IV=0.76 dl.g⁻¹; Mw=101 kD) supplied by Purac.
- Ester terminated poly-DL-lactide Purasorb PDL-05 (IV=0.50 dl.g⁻¹; Mw=62 kD) supplied by Purac.
- Sodium alendronate, supplied by Polpharma S A, ground to a D90 particle size of less than 25 µM.
- Poly(vinyl alcohol), supplied by Sigma Aldrich, 87 to 89% hydrolysed with a molecular weight of 13 to 124 kD (product code 363170).
- Dichloromethane, HPLC grade, supplied by BDH Prolabo VWR.
- Sodium chloride, Ph Eur grade, supplied by BDH Prolabo VWR.
- Deionised water, purified using a purifier available from Veolia Water Solutions & Technologies S A under the trade mark ELGA Purelab Option Q DV25.

These materials were used to prepare alendronate encapsulated polymer particles with a targeted drug content of 9% using a solid-oil-water emulsion method, as follows.

1.00 g polymer was dissolved in 4 ml dichloromethane (DCM). 100 mg sodium alendronate was added to the solution. The suspension was homogenised with a vortex mixer at the highest speed until the polymer dissolved.

The resulting suspension was slowly injected from a 10 ml glass syringe into a solution of 0.1% w/w poly(vinyl alcohol) (PVA) and 4% w/w sodium chloride while stirring with a magnetic stirrer.

The suspension of polymer particles in water was then homogenised using an IKA T25 rotor-stator homogeniser at 6400 rpm and further stirred with a magnetic stirrer under conditions in which the DCM solvent could evaporate.

The polymer particles were separated from the liquid phase by centrifuging and filtration, and then washed with deionised water. The polymer particles were then lyophilised.

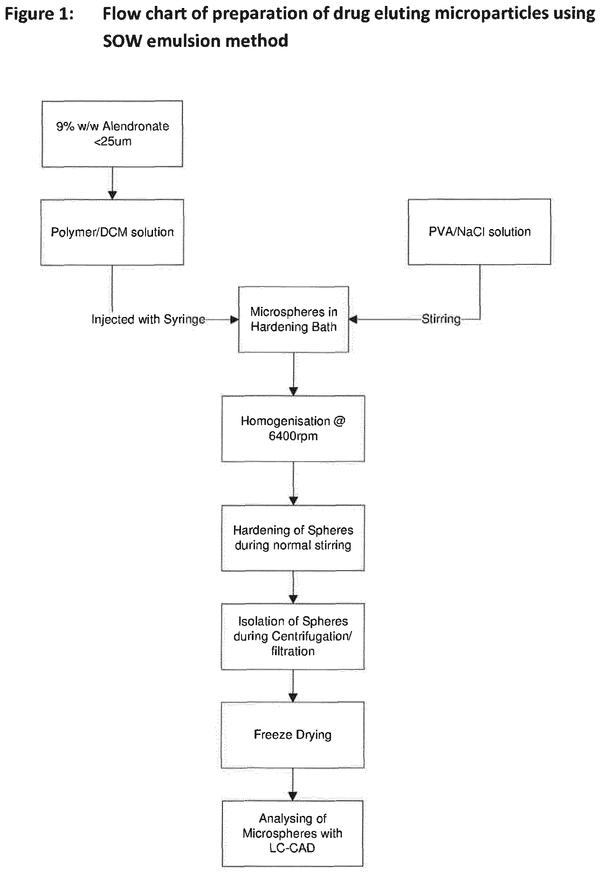

The steps of the solid-oil-water method are depicted graphically in Figure 1.

The drug encapsulation efficiency and particle size of the polymer particles were evaluated as follows:

### Measurement of drug encapsulation efficiency

### HPLC

Chromatographic analysis is carried out on an Agilent 1200 HPLC system (Agilent) with Corona Charged Aerosol Detector (ESA) to measure sodium alendronate content encapsulated into the polymer particles. The mobile phases used are 5% acetonitrile (BDH Prolabo VWR) in deionised water (ELGA, Purelab Option Q DV25) as mobile phase A, and 5% acetonitrile in deionised water with 0.03% trifluoroacetic acid (Sigma Aldrich) as mobile phase B. The gradient increased linearly from 30% B to 100% B in 5 mins with a hold time of 2 mins. The flow rate is 0.5 ml.min⁻¹ with an injection volume of 10 µl. Separation is carried out on a column measuring 3.2 × 50 mm, thickness 5 µm, as supplied by Siele Technologies under the trade mark Primesep SB. The column temperature is maintained at 40°C. Standards are made in duplicate to a concentration of 0.5 mg.ml⁻¹ sodium alendronate (Polpharma SA) in diluent (mobile phase A). Samples are prepared by sonicating the polymer particles with dichloromethane (BDH Prolabo VWR) to dissolve the polymer. Deionised water is then added to dissolve the sodium alendronate. Sodium alendronate is not soluble in DCM, and water and DCM are not miscible, which causes two sample layers to form, where the top layer contains water and sodium alendronate. A sample from the top layer is centrifuged at 3500 rpm for 5 mins (Clifton), and the supernatant filtered into HPLC vials for analysis.

### UV/visible spectrophotometry

A sodium alendronate solution (10625 mg.ml⁻¹) is made using 162.5 mg of alendronate sodium which is weighed into a 100 ml volumetric flask. The flask is filled approximately half full with deionised water and the solution is heated in a water bath set at 40°C until all of the alendronate has dissolved (approximately 5 to 10 minutes). The solution is made up to volume with deionised water and cooled to room temperature. Once cooled, the solution is topped up to volume with deionised water if and as if required.

A derivatisation reagent (5.5 mM CuSO₄, 3 mM HNO₃) is made using 0.8778 g of CuSO₄ and 0.19 ml 70% nitric acid, made up using deionised water in a 1000 ml volumetric flask.

Calibration solutions are made by transferring aliquots of 1.0, 3.0, 5.0 and 10.0 ml of the alendronate solution into 100 ml volumetric flasks. 50 ml of derivatisation reagent is added to the flasks, and the solutions are topped up to volume with deionised water. Final concentrations of calibration solutions will be 0.01625, 0.04875, 0.08125 and 0.1625 mg.ml⁻¹. A blank calibration solution is made in the same way, using the derivatisation solution and water.

A 55 mg sample of alendronate/polymer particles to be assayed is sonicated with 4.0 ml of dichloromethane for 15 minutes. It is sonicated for a further 5 minutes after addition of 10 ml of deionised water. A 5.0 ml sample of the top water layer is drawn off and transferred to a centrifuge tube, and is then centrifuged at 3500 rpm for five minutes. A 2.0 ml sample of the supernatant is transferred into a vial and reacted with 2.0 ml of the derivatisation agent.

A calibration curve created by measuring absorbance at 235 nm. The alendronate concentration in the sample is derived from the calibration curve

### Particle size measurement of drug encapsulated particles

The particles size was measured using the HELOS & RODOS (Sympatec GmbH) laser diffraction particle size analyser (PSA). The particles were placed on the VIBRI shoot and analysed with the RODOS dry dispersion method. Every test is repeated three times. The material on the front of the shoot is transported into the dispersion funnel with a feed rate of 30% and a pressure of 0.25 MPa (2.5 bar). An air flow measurement was carried out as a reference before each analysis. The result of the measurements were analysed using the Frauenhofer equation by the particle size analyser to calculate the particles size.

The results obtained are given in the table below:

| Polymer | Encapsulation efficiency (%) | Particle size D50 (µm) |
|---|---|---|
| PDLG-5004A | 70 | 35 |
| PDLG-5002A | 57 | 64 |
| PDLG-7502A | 41 | 30 |
| PDL-02A | 69 | 42 |

Control particles were prepared using the same method but omitting the sodium alendronate.

### Calcium phosphate cement preparation

Four powder blends were prepared as follows:
I: Control (calcium phosphate cement with no drug and no polymer): 9.75 g β-tricalcium phosphate powder (supplied by Plasma Biotal Ltd) with a particle size D90 of less than 25 µm was blended with 0.25 g sodium pyrophosphate (supplied by Alfa Aesar GmbH) using a powder blender. The powder blender was operated for a period of between 10 and 90 minutes, at a blending speed of between 35 and 90 rpm, until the powders were fully mixed. The mixing conditions depend on the total mass of the powder mixture.
II: Calcium phosphate cement with drug: 9.7 g β-tricalcium phosphate powder with a particle size D90 of less than 25 µm was blended with 0.25 g sodium pyrophosphate and 0.05 g sodium alendronate (supplied by Polpharma SA) using the powder blender.
III: Control (calcium phosphate cement with drug-free polymer particles): 8.587 g β-tricalcium phosphate powder with a particle size D90 of less than 25 µm was blended with 0.25 g sodium pyrophosphate and 1.163 g blank drug-free polymer particles using the powder blender.
IV: Calcium phosphate cement with drug encapsulated polymer particles: 8.587 g β-tricalcium phosphate powder with a particle size D90 of less than 25 µm was blended with 0.25 g sodium pyrophosphate and 1.163 g alendronate encapsulated particles using the powder blender.

The powder blends were mixed with aqueous solution of 4M orthophosphoric acid (Sigma-Aldrich) and 0.1M sulphuric acid (Sigma-Aldrich). Details of this approach are disclosed in US-6018095. The product is a resorbable calcium phosphate, dicalcium phosphate dihydrate (brushite). The ratio of liquid-to-powder ratio was 0.5 ml.g⁻¹ and the blend was mixed using a spatula for 30 to 60 seconds to allow the mixture to transform from milky form to a paste. A portion of the paste was placed in a 10 ml syringe and the remainder was retained for measurement of the final setting time.

Setting times were measured in accordance with ASTM C66-99 using Gillmore Needle Apparatus (supplied by Labquip Projects Ltd) to determine the initial and final setting times (tᵢ and t_{f}). The apparatus consists of a light needle of mass 113.4 ± 0.5 g and needle tip diameter of 2.12 ± 0.05 mm for determining tᵢ and a heavy needle of mass 453.6 ± 0.5 g and needle tip diameter of 1.06 ± 0.05 mm for determining t_{f}.

A 10 g powder batch was mixed with 5 ml liquid for 1 minute. A maximum of 4 minutes was allowed for manual application into PTFE moulds possessing three specimen cylinders of height 6 mm and diameter 12 mm. The mould was placed in an oven at 37°C to represent the clinical environment. The cement specimen was tested every minute by placing the needles on to the cement surface. Initial setting time is defined as the time when the cement specimen will bear the weight of the lighter needle without appreciable indentation. Final setting time is when the specimen bears the weight of the heavier needle without appreciable indentation.

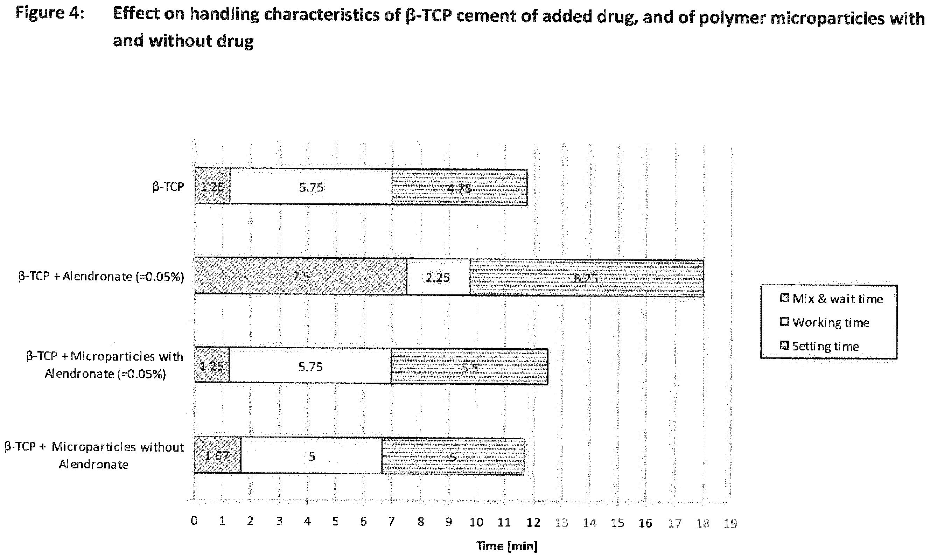

The cement was extruded from the syringe on to a glass block in set intervals of 15 to 45 seconds depending on the current stage of the total handling characteristics. The start of the working time period was recorded once the cement showed toothpaste like consistency and the time prior to this stage is called the mixing and waiting time. The working time period for a cement material starts when the consistency of the material is such that the material does not run freely and is largely self-supporting when extruded on to the glass surface in a quantity of about 0.3 to 0.5 ml. The end of the working time period is reached when the consistency of the cement is such that it is no longer possible to extrude the cement manually from a syringe through a cannula having a diameter of about 2 mm. This marks the beginning of the setting time period, as shown in Figure 4.

### EXAMPLE 2 - Water-oil-oil emulsion method

### Preparation of particles

The following materials were used to manufacture particles:
- Ester terminated lactide/glycolide copolymer Purasorb PDLG-5004 (IV=0.41 dl.g⁻¹; Mw=42 kD) supplied by Purac.
- Sodium alendronate, supplied by Polpharma S A, ground to a D90 particle size of less than 25 µm.
- Liquid paraffin, supplied by Merck.
- Sorbitane trioleate surfactant, supplied by SigmaAldrich under the trade mark Span 85.
- n-hexane, supplied by Fisher Scientific.
- Dichloromethane, HPLC grade, supplied by BDH Prolabo VWR.
- Acetonitrile, supplied by Acros Organics.

50 mg of alendronate sodium was weighed into a small glass vial. A quantity of deionised water (see the table below) was added to the glass vial and gently agitated. 0.4% w/v poly(vinyl alcohol) was added as the emulsifier to the water phase for formation of primary emulsion. The alendronate solution was heated to 40 to 50°C to dissolve te alendronate. 250 mg of PLGA copolymer (PDLG 5004) was weighed into a glass syringe (with lid on) and dissolved in a 1:1 mixture of dichloromethane and acrylonitrile. The syringe was gently agitated to dissolve the polymer. The aqueous alendronate solution was added to the syringe containing the polymer solution and homogenised at 1750 g (14400 rpm) for one minute to form the primary W-O₁ emulsion.

The primary emulsion W-O₁ was added to a mixture of paraffin and sorbitane trioleate surfactant (96:4 w/w) which provided the(second non aqueous phase (O₂) and homogenised twice to form the secondary emulsion (W-O₁-O₂). After 30 minutes, the resultant emulsion was stirred on a magnetic stirrer to allow evaporation of solvents. After stirring, the emulsion was centrifuged. The precipitate was washed eight times with 15 ml portions of n-hexane each time and centrifuged at designated speed to wash off the paraffin. The resultant precipitate was dispersed in 2 ml of n-hexane and evaporated in air overnight to obtain the microparticles.

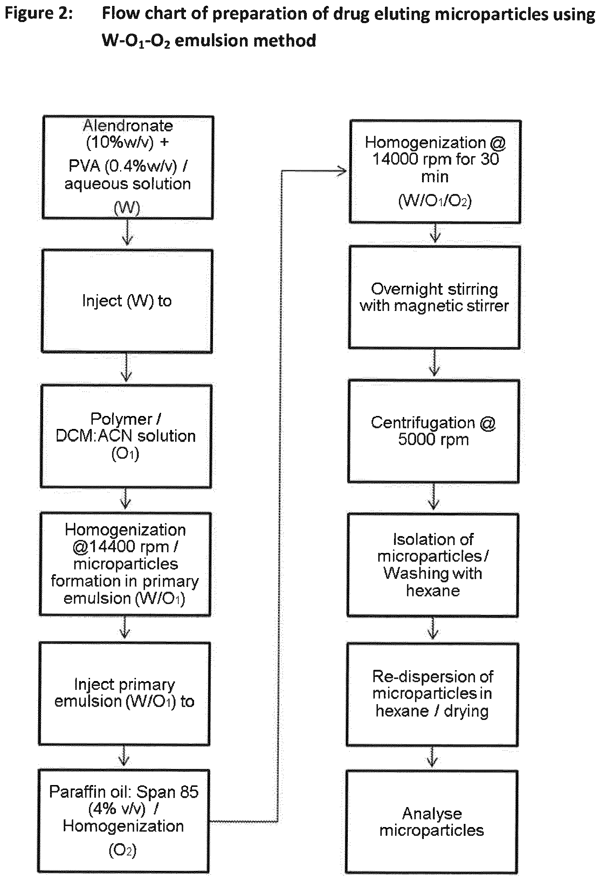

The steps of the water-oil-oil method are depicted graphically in Figure 2.

### Process conditions

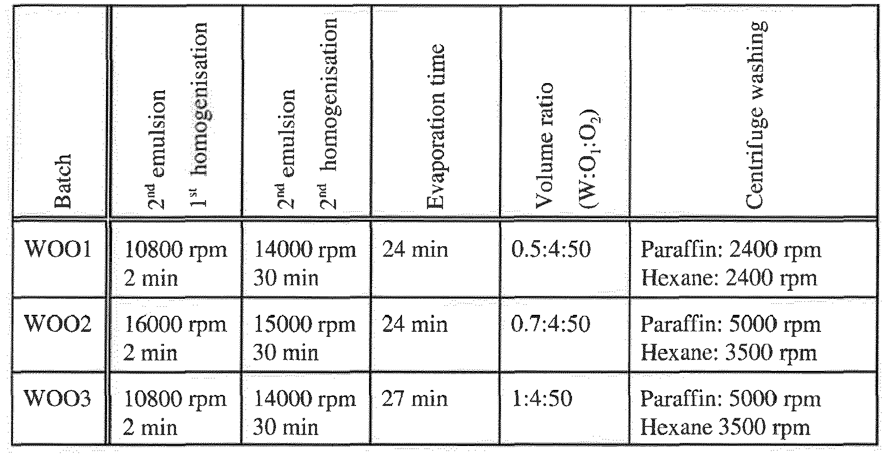

### Particle properties

| Batch | Encapsulation efficiency (%) | Particle size (µm) |
|---|---|---|
| WOO1 | 72 | x₁₀ -1.65 |
| | | x₅₀ -18.35 |
| | | x₉₀ -141.79 |
| WOO2 | 57 | x₁₀ -2.095 |
| | | x₅₀ -60.245 |
| | | x₉₀ -159.6 |
| WOO3 | 89 | x₁₀-3.44 |
| | | x₅₀ -29.335 |
| | | x₉₀ -110.07 |

### EXAMPLE 3 - Melt processing

### Preparation of particles

The following materials were used to manufacture particles:
- Ester terminated L-lactide/DL-lactide copolymer (70:30) Resomer LR706 (IV=4.0 dl.g⁻¹ (3.3 to 4.2 dl.g⁻¹)) supplied by Boehringer Ingelheim.
- Poly(ε-caprolactone) Resomer C (IV=1.0 dl.g⁻¹) supplied by Boehringer Ingelheim.
- Acid terminated lactide/glycolide copolymer Purasorb PDLG-5004A (IV=0.41 dl.g⁻¹; Mw=53 kD) supplied by Purac.
- Sodium alendronate, supplied by Polpharma S A, ground to a D90 particle size of less than 25 µm.

Drug containing fibres were manufactured using a twin screw extruder (Leistritz type ZSE 18 HP-40D) with die diameter of 3 mm. The screws have a diameter of 18 mm and the screw length/diameter ratio is 40. The drug was added to the polymer in a weight proportion 1:4 drug:polymer. Samples were manufactured from each of the polymers mentioned above. The extrusion temperatures for the three polymers were 170 to 175 °, 70 to 75°, and 100 to 110°C, respectively

The polymer drug mixtures were homogenized in a shaker (RETSCH, AS 200 basic) and dried in a vacuum oven (<5 mbar) at 40°C for 24 hours.

Fibres are produced by using the required die diameter or through drawing of the extruded fibre out of the die. Melt spinning can be used to produce thinner fibres.

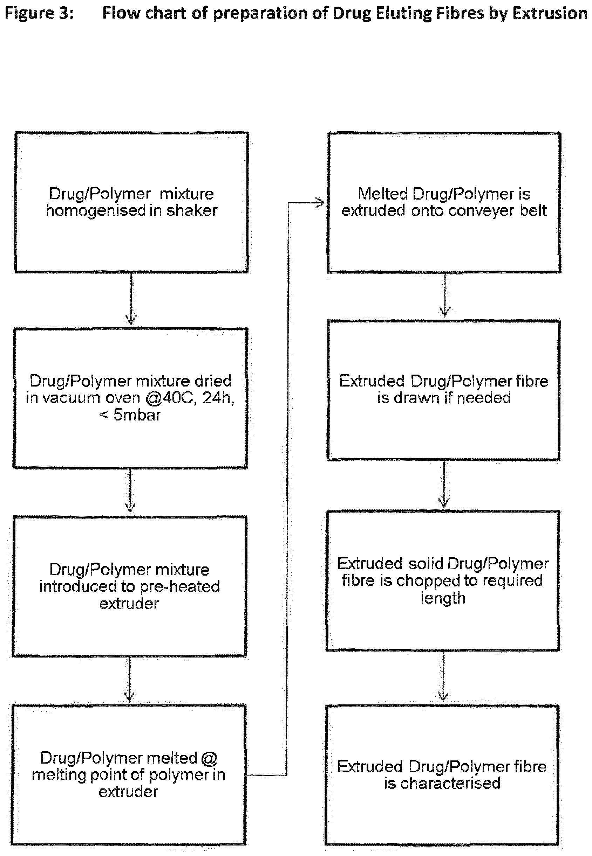

The steps of the melt processing method are depicted graphically in Figure 3.

### Particle properties

The alendronate concentrations in the particles was determined using the UV-visible spectrophotometry method, as follows:

| Batch | Encapsulation efficiency (%) |
|---|---|
| Poly(L-DL)lactide | 78 |
| Poly(caprolactone) | 69 |
| PLGA | 55 |

Clause 5: A formulation of any one of clauses 1 to 4, in which the weight proportion of the bisphosphonate expressed as a proportion of the weight of the formulation is at least about 0.5%.
Clause 6: A formulation of any one of clauses 1 to 5, in which the particles of the bisphosphonate which are embedded in the particles of the polymeric material have a particle size of not more than about 70 µm.
Clause 7: A formulation of any one of clauses 1 to 6, in which the polymeric material is hydrophobic.
Clause 8: A formulation of any one of clauses 1 to 7, in which the polymeric material comprises a monomer which has hydrophobic properties and a monomer which has hydrophilic properties.
Clause 9: A formulation of any one of clauses 1 to 8, in which the polymeric material chains are terminated with hydrophilic species.
Clause 10: A formulation of any one of clauses 1 to 9, in which the polymeric material is semi-crystalline.
Clause 11: A formulation of any one of clauses 1 to 10, in which the polymeric material is amorphous.
Clause 12: A formulation of any one of clauses 1 to 11, in which the polymeric material includes a lactide polymer.
Clause 13: A formulation of any one of clauses 1 to 11, in which the polymeric material is a lactide glycolide copolymer.
Clause 14: A formulation of any one of clauses 1 to 13, in which the polymeric material has a molecular weight of at least about 15 kD.
Clause 15: A formulation of any one of clauses 1 to 14, in which the polymeric material has a molecular weight of not more than about 200 kD.
Clause 16: A formulation of any one of clauses 1 to 15, in which the particles of the polymeric material are in the form of fibres.
Clause 17: A formulation of clause 16, in which the fibres are formed by extrusion.
Clause 18: A bone filler composition which comprises a mixture of a curable calcium phosphate based bone filler which is formed from a liquid component and a calcium phosphate based powder component, and a formulation of any one of the preceding clauses.
Clause 19: The composition of clause 18, in which the powder component comprises at least 50% by weight β-tricalcium phosphate, expressed as a percentage of the total weight of the powder component of the bone filler.
Clause 20: The composition of clause 18 or clause 19, in which the bisphosphonate loaded polymeric material particles are present in an amount of not more than about 50% by weight, expressed as a percentage of the total weight of powder component of the bone filler.
Clause 21: The composition of any one of clauses 18 to 20, in which the bisphosphonate loaded polymeric material particles are present in an amount of at least about 1% by weight, expressed as a percentage of the total weight of powder component of the bone filler.

## Claims

1. A composition comprising a calcium phosphate based powder component and a bisphosphonate in particulate form, the composition forming a calcium phosphate based bone filler when mixed with a liquid component, and in which the particles of the bisphosphonate are embedded in particles of a polymeric material which resorbs when the bone filler is implanted.

2. The composition as claimed in claim 1, in which the particles of the bisphosphonate are embedded in the polymeric material using a melt processing technique.

3. The composition as claimed in claim 1 or claim 2, in which 90% by weight of the particles of the polymeric material have a particle size of not more than about 100 µm.

4. The composition as claimed in any of claims 1 to 3, in which the polymeric material is hydrophobic

5. The composition as claimed in any of claims 1 to 4, in which the polymeric material comprises a monomer which has hydrophobic properties and a monomer which has hydrophilic properties.

6. The composition as claimed in any of claims 1 to 5, in which the polymeric material chains are terminated with hydrophilic species.

7. The composition as claimed in any one of claims 1 to 6, in which the polymeric material is semi-crystalline.

8. The composition as claimed in any one of claims 1 to 7, in which the polymeric material is amorphous.

9. The composition as claimed in any one of claims 1 to 8, in which the polymeric material includes a lactide polymer.

10. The composition as claimed in any one of claims 1 to 9, in which the polymeric material is a lactide glycolide copolymer.

11. The composition as claimed in any one of claims 1 to 10, in which the polymeric material has a molecular weight of at least about 15 kD.

12. The composition as claimed in any one of claims 1 to 11, in which the polymeric material has a molecular weight of not more than about 200 kD.

13. The composition as claimed in any one of claims 1 to 12, in which the particles of the polymeric material are in the form of fibres.

14. The composition as claimed in claim 13, in which the fibres are formed by extrusion.

## Patentansprüche

1. Zusammensetzung, umfassend eine Pulverkomponente auf Basis von Calciumphosphat und ein Bisphosphonat in Teilchenform, wobei die Zusammensetzung ein Knochenfüllmittel auf Basis von Calciumphosphat bildet, wenn sie mit einer flüssigen Komponente vermischt wird, und in welcher die Teilchen des Bisphosphonats in Teilchen eines polymeren Materials, welches resorbiert, wenn das Knochenfüllmittel implantiert wird, eingebettet sind.

2. Zusammensetzung nach Anspruch 1, in welcher die Teilchen des Bisphosphonats in dem polymeren Material unter Verwendung einer Schmelzverarbeitungstechnik eingebettet sind.

3. Zusammensetzung nach Anspruch 1 oder Anspruch 2, in welcher 90 Gewichts-% der Teilchen des polymeren Materials eine Teilchengröße von nicht mehr als etwa 100 µm aufweisen.

4. Zusammensetzung nach einem der Ansprüche 1 bis 3, in welcher das polymere Material hydrophob ist.

5. Zusammensetzung nach einem der Ansprüche 1 bis 4, in welcher das polymere Material ein Monomer, welches hydrophobe Eigenschaften aufweist, und ein Monomer, welches hydrophile Eigenschaften aufweist, umfasst.

6. Zusammensetzung nach einem der Ansprüche 1 bis 5, in welcher die polymeren Materialketten mit hydrophilen Spezies terminiert sind.

7. Zusammensetzung nach einem der Ansprüche 1 bis 6, in welcher das polymere Material semikristallin ist.

8. Zusammensetzung nach einem der Ansprüche 1 bis 7, in welcher das polymere Material amorph ist.

9. Zusammensetzung nach einem der Ansprüche 1 bis 8, in welcher das polymere Material ein Lactid-Polymer einschließt.

10. Zusammensetzung nach einem der Ansprüche 1 bis 9, in welcher das polymere Material ein Lactid-Glycolid-Copolymer ist.

11. Zusammensetzung nach einem der Ansprüche 1 bis 10, in welcher das polymere Material ein Molekulargewicht von mindestens etwa 15 kD aufweist.

12. Zusammensetzung nach einem der Ansprüche 1 bis 11, in welcher das polymere Material ein Molekulargewicht von nicht mehr als etwa 200 kD aufweist.

13. Zusammensetzung nach einem der Ansprüche 1 bis 12, in welcher die Teilchen des polymeren Materials in Form von Fasern vorliegen.

14. Zusammensetzung nach Anspruch 13, in welcher die Fasern durch Extrusion gebildet sind.

## Revendications

1. Composition comprenant un composant pulvérulent à base de phosphate de calcium et un bisphosphonate sous forme particulaire, la composition formant un agent de remplissage osseux à base de phosphate de calcium lorsqu'elle est mélangée avec un composant liquide, et dans laquelle les particules de bisphosphonate sont noyées dans des particules d'un matériau polymère qui se résorbe lorsque l'agent de remplissage osseux est implanté.

2. Composition selon la revendication 1, dans laquelle les particules de bisphosphonate sont noyées dans le matériau polymère à l'aide d'une technique de traitement par fusion.

3. Composition selon la revendication 1 ou la revendication 2, dans laquelle 90 % en poids des particules du matériau polymère ont une taille de particules non supérieure à environ 100 µm.

4. Composition selon l'une quelconque des revendications 1 à 3, dans laquelle le matériau polymère est hydrophobe.

5. Composition selon l'une quelconque des revendications 1 à 4, dans laquelle le matériau polymère comprend un monomère qui a des propriétés hydrophobes et un monomère qui a des propriétés hydrophiles.

6. Composition selon l'une quelconque des revendications 1 à 5, dans laquelle les chaînes du matériau polymère sont terminées par des espèces hydrophiles.

7. Composition selon l'une quelconque des revendications 1 à 6, dans laquelle le matériau polymère est semi-cristallin.

8. Composition selon l'une quelconque des revendications 1 à 7, dans laquelle le matériau polymère est amorphe.

9. Composition selon l'une quelconque des revendications 1 à 8, dans laquelle le matériau polymère comprend un polymère de lactide.

10. Composition selon l'une quelconque des revendications 1 à 9, dans laquelle le matériau polymère est un copolymère de lactide-glycolide.

11. Composition selon l'une quelconque des revendications 1 à 10, dans laquelle le matériau polymère a une masse moléculaire d'au moins environ 15 kD.

12. Composition selon l'une quelconque des revendications 1 à 11, dans laquelle le matériau polymère a une masse moléculaire non supérieure à environ 200 kD.

13. Composition selon l'une quelconque des revendications 1 à 12, dans laquelle les particules du matériau polymère sont sous la forme de fibres.

14. Composition selon la revendication 13, dans laquelle les fibres sont formées par extrusion.
